# EUROPEAN PATENT SPECIFICATION

(11) **EP 1 052 946 B1**
(45) Date of publication and mention of the grant of the patent: **07.04.2004**
(21) Application number: 99902682.6
(22) Date of filing: 27.01.1999
(51) Int. Cl.: A61B 17/80

(54) **A FIXATION DEVICE FOR A BONE FRACTURE**
VORRICHTUNG ZUR FIXIERUNG VON KNOCHENBRÜCHEN
DISPOSITIF DE FIXATION D'UN OS FRACTURE

(30) Priority: 31.01.1998 GB 9801986
(43) Date of publication of application: 22.11.2000
(73) Proprietor: DEPUY INTERNATIONAL LIMITED, Beeston, Leeds LS11 8DT (GB)
(72) Inventor: FITZPATRICK, David Paul, York YO1 3UB (GB); PASCAUD, Raphael Stephane, Boroughbridge YO51 9LH (GB)
(74) Representative: Belcher, Simon James
(86) International application number: PCT/GB1999/000280
(87) International publication number: WO 1999/038447

(56) References cited:
- EP-A- 0 014 823
- EP-A- 0 024 635
- EP-A- 0 295 041
- BG-A- 100 203
- FR-A- 2 416 683
- GB-A- 1 517 161
- US-A- 5 702 396

## Description

This invention relates to a fixation device for a bone fracture. The device comprises a plate which is arcuate when viewed along its length and is particularly suitable for use with a bone that is generally elongate so that the plate can be fitted around the bone.

EP-A-24635 discloses an internal fixation device for a bone fracture. It comprises a metallic plate having an arcuate shape so that it can be positioned around the fractured bone, and internally projecting teeth formed on two edges. It can be secured to a bone at a fracture site by deforming the plate inwardly to force the teeth to engage the bone.

The generic document EP-A-295 041 also discloses such a fixation device further having bone engaging teeth on its body portion.

Contact between the bone and the internal surface of the plate in the longitudinally extending central region of the plate can stabilise the connection between the bone and the plate. However, this can restrict access to the bone by body fluids which can lead to undesirable degradation of the bone tissue.

The present invention provides a fracture fixation device which has a plurality of inwardly extending depressions formed in its body portion of which at least can engage the surface of the bone to maintain a gap between the bone and the internal surface of the body portion in regions of the plate around the depressions.

Accordingly, in one aspect, the invention provides a fixation device for a bone fracture, which comprises a plate which is arcuate when viewed along its length so that it can be fitted around the bone in the region of the fracture, the plate having a body portion and an array of inwardly directed fastening projections along each of its opposite edges which can fixedly engage the bone along its length when the plate is positioned around the bone and its edges are subjected to an inward clamping force, the device having a plurality of inwardly extending depressions formed in its body portion of which at least some can engage the surface of the bone to maintain a gap between the bone and the internal surface of the body portion in regions of the plate around the depressions.

The provision of the depressions in the body portion can reduce the area of bone which is contacted by the plate. This has the consequential advantage that degradation of the bone tissue due to restricted access of body fluids to the bone tissue can be minimised.

Furthermore, the contact between the plate and the bone tissue provided by the depressions can approximate to point-to-surface contact by selection of a suitable configuration of depressions, in particular when compared with the surface-to-surface in the case of a plate without the depressions. Such contact can provide enhanced stability of the plate with respect to the bone. Preferably, the depressions have a cutting edge which can form an indented impression in the surface of the bone when pressed against the surface. This has the advantage of enhancing the stability of the plate with respect to the bone effectively. For example, at least some, and preferably each, of the depressions can have a sharpened edge. In cases where the depression is not perforated, the edge can be a point.

The number and arrangement of the depressions which contact the bone will depend in particular on the configuration of the surface of the bone against which the plate is to be fitted and the position of the bone on the plate. It may be that, in some applications, no more than one or two of the depressions will contact the bone. In order to maximise the stability of the plate relative to the bone, it can be preferable to position the plate so as to maximise the number of the depressions that contact the bone, subject of course to satisfactory positioning of the plate relative to the fracture that is to be treated.

A preferred arrangement of the depressions can comprise two or more spaced apart linear arrays, the arrays extending along the device parallel to the axis thereof. For example, one linear array can be provided along the device, one on each side of a centre line.

It can be preferred for at least some of the depressions to be perforated. The perforation of the depressions can provide an edge that can form an indented impression in the surface of the bone when pressed against the surface. By selection of an appropriate configuration, a perforated depression can accommodate a fastener such as a screw by which the plate can be fixed to the bone in its body portion. If the depression is one which is in contact with the bone. the tightening of a fastener such as a screw can urge the depression, together with other surrounding depressions, into contact with the bone, so as to form an indented impression in the surface of the bone. Some or all of the perforations in the depressions can be elongate, for example generally oval when they are rounded, so that the orientation of the fastener within the depression can be selected according to the requirements of a particular application. For example, an inclined fastener such as a screw can be used to apply a force in a direction along the axis of a bone. Two inclined fasteners might therefore be used with a fixation device to apply a compressive force to a bone across a fracture site.

The shape of the depressions will be selected according to factors which include providing appropriate contact with the bone, and maintaining the ability of the plate to withstand stresses without deformation. Rounded depressions can be preferred for many constructions of the device. Rounded depressions can minimise adverse effect on the ability of the device to withstand applied stresses. With an appropriate size, they can also conveniently accommodate fasteners such as screws when perforated.

The technique used to form the depressions will depend on factors which include the technique used to form the plate and the configuration of the depressions. Preferably, the projections are formed by a punching operation so that there is a recess in the plate opposite to each projection on the surface that faces away from a bone when the fixation device is in use. The use of a punching operation has the advantage that it can create a cutting edge on the depression where it is to contact the bone. It can result in the formation of a recess in the plate opposite to each projection, which can accommodate at least partially a head of a fastener, especially when the head has a countersunk configuration. A further advantage of use of a punching operation is that it can be performed easily and conveniently.

Alternative techniques for forming the depression might include for example fixing a depression to the inwardly facing surface of the plate. For example, a quantity of a settable fluid (for example a curable polymeric composition or a molten metallic material) might be placed on the plate to harden *in situ* to form the depressions, or a shaped depression might be affixed to the plate, for example by bonding.

A cutting edge can be formed on the depression by grinding. When the depression is circular, the cutting edge can be formed by means of a rotating grinding head.

Preferably, the fastening projections on the device are arranged so that lines extending along respective edges of the plate joining adjacent fastening projections each have a wavy configuration. This feature is disclosed and claimed in WO-A-99/38448. The arrangement of fastening projections on the edges of the plate in lines which have a wavy configuration can reduce the tendency for the projections to create a longitudinally extending line of weakness in a bone when the plate has been deformed inwardly to cause the projections to engage the bone, compared with a device such as the one disclosed in EP-A-24635 in which a line joining the bone-engaging teeth on each edge is straight. The significance of this advantage will be readily appreciated.

The wavy configuration of the line which joins the fastening projections will generally be such that the line crosses a reference line extending generally along the device following the configuration of the body portion at several points. When the device of the invention is essentially straight for use on a straight bone, the reference line will be essentially straight. The device of the invention might however be curved for use on a curved bone, in which case the reference line will be curved. The degree of the waviness of the line which joins the projections will depend on the length of the device and the spacing between the projections. It can be preferred for the projections to be spaced substantially evenly along the edges of the device. Preferably, the wavy configuration of the line which joins the projections is substantially even along the length of the device. Preferably, the ratio of the distance between adjacent troughs or peaks to the spacing between the projections is approximately equal to an integer. For example, the ratio might have a value of 3, 4 or more. Preferably, the ratio of the distance between adjacent troughs to the spacing between the projections is about 2.

Preferably, the plate comprises two arrays of fastening fingers arranged along the said opposite edges of the plate, the fastening projections being provided on the fastening fingers. The fastening fingers will each extend from the axially extending spine provided by the body section of the plate so that they can be deformed inwardly individually to cause the fastening projections to engage the bone. This arrangement has the advantage of facilitating the inward deformation of the plate, allowing the plate to be deformed inwardly appropriately at spaced apart points along its length to accommodate the configuration of the bone that is being treated.

The fastening fingers can be tapered inwardly along their lengths towards their ends. This has the advantage of minimising concentration of stress at the root of the finger where it is joined to the spine of the body portion of the device, leaving the fingers flexible towards their ends so that inward deformation of fingers in the region towards their ends is facilitated.

The wavy configuration of the line joining adjacent fastening projections can be provided by appropriate selection of the length of the fastening fingers. It can be preferred for the fastening fingers in each array are arranged in first and second sets, each fastening finger of the first set having a fastening finger of the second set on each side, the length of each fastening finger of the first set being less than the length of each of the adjacent fastening fingers of the second set. The ratio discussed above of the distance between adjacent troughs or peaks to the spacing between the projections can be selected by appropriate selection of the numbers of fingers in the first and second sets. For example, the ratio can have a value of 2 when there are equal numbers of fingers in the first and second sets. with one fixation finger of the second set between each pair of adjacent fastening fingers of the first set.

Preferably, the lengths of fastening fingers of the first set or the second set or, especially both sets. are approximately equal.

The arcuate cross-sectional shape of the device will be selected according to the size and nature of the bone on which the device is to be used. It will generally be desirable for the plate to extend at least half way around the bone. The arcuate shape need not be circular; whether or not it is circular, it is preferred that it extend through an angle of between about 150° and 210° so that the points at which the fastening projections engage the surface of the bone are approximately diametrically opposite to one another. The fastening projections on the opposite edges of the plate will then extend approximately directly towards one another.

The device will generally be made from a single plate of one material, although devices formed from two or more pieces, of a common material or different materials. The material(s) of the plate will be selected according to the physical requirements placed on the device when in use and to the requirements for compatibility with materials with which the device will come into contact when in use. The material will generally be metallic. Biocompatible titanium based alloys, stainless steels, and cobalt chromium based steels might be suitable for some applications. Polymeric materials might be useful for some applications, in particular in which the stresses to which the device is exposed in use are small. Materials which are able to be resorbed after implantation might be useful for some applications.

The techniques used for making the device will depend on factors such as its configuration and the materials being used. The device might be made using a moulding or casting operation. It might be made by forming a sheet substrate. Fastening fingers might then be formed by cutting. Fastening projections can be formed by bending when they are formed integrally with the body portion of the device. The fastening projections are preferably sharpened to facilitate the formation of indented impressions when they engage the surface of the bone.

The technique used to form the depressions will depend on factors which include the technique used to form the plate and the configuration of the depressions. Preferably, the projections are formed by a punching operation to that there is a recess in the plate opposite to each projection. The use of a punching operation has the advantage that is can create a cutting edge on the depression where it is to contact the bone. It can result in the formation of a recess in the plate opposite to each projection, which can accommodate at least partially a head of a fastener, especially when the head has a countersunk configuration. A further advantage of use of a punching operation is that it can be performed easily and conveniently.

The device of the invention can be applied to the long bones of the leg and arm. It finds particular application in bridging a fracture of the femur towards its proximal end, in particular in the case of a femur in which a femoral stem component of a hip prosthesis has been implanted, which has fractured at about the bottom of the stem. A similar application would be in the case of a fractured humerus.

The present invention will now be described, by way of example only, with reference to the accompanying drawings, in which:
Figure 1 is a plan view of a fixation device according to the present invention.
Figure 2 is a side view of the fixation device shown in Figure 1.
Figure 3 is an end view of the fixation device shown in Figure 1.
Figure 4 is an enlarged cross-sectional view through the fixation device shown in Figure 1, on the line A-A.
Figure 5 is a transverse view, partially in section, through the device shown in Figure 1 in place on a bone.

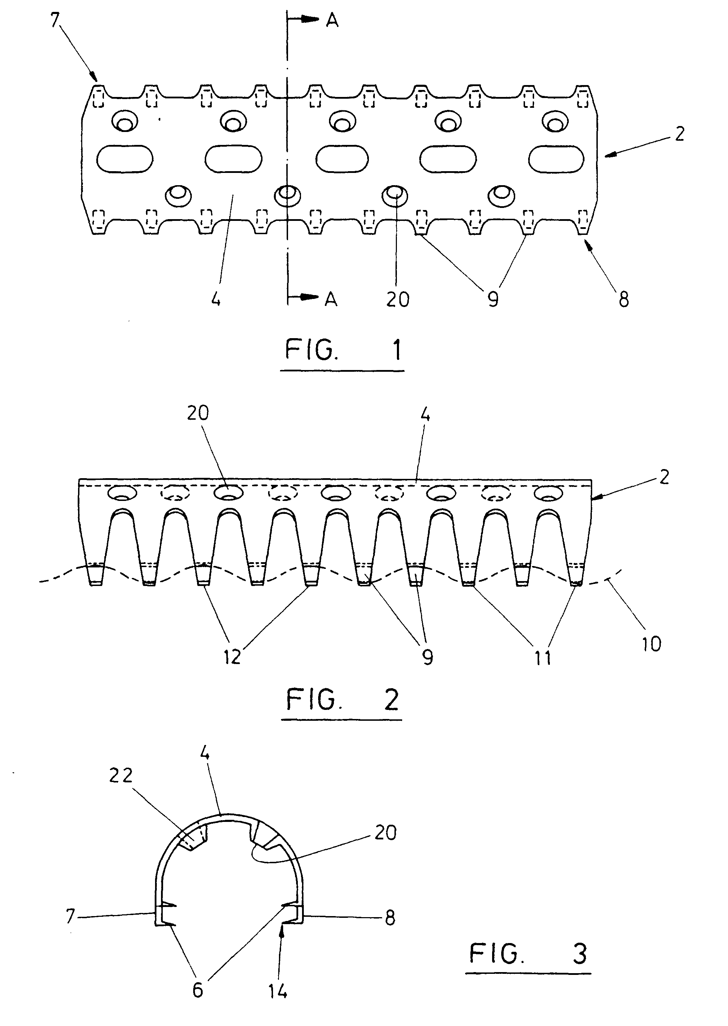

Referring to the drawings, which show a fixation device 2 which is formed from a titanium based alloy or a stainless steel. The device is generally arcuate when viewed along its length (as can be seen in Figure 3). It has a body portion 4 and an array of inwardly directed fastening projections 6 along each of its opposite edges 7, 8. Each of the fastening projections is provided on a fastening finger 9 which is tapered inwardly along its length (as can be seen in Figure 2), being relatively wide at its root and no wider than the fastening projection 6 itself that is provided at its tip.

As can be seen in Figure 3, the fastening projections 6 are arranged on the fastening fingers 9 so that the projections on opposite sides of the device are directed towards one another.

The lengths of the fastening fingers 9 alternate along the length of the fixation device so that a line 10 joining the fastening projections 6 has a wavy configuration (as can be seen in Figure 2. The alternating lengths of the fastening fingers means that the fingers can be divided into first and second sets 11, 12, with each fastening finger of the first set having a fastening finger of the second set on each side. Because there are only first and second sets of fastening fingers in the embodiment shown in the drawings, fastening fingers of the second set are immediately adjacent to a fastening finger of the first set. If there were a third length of fastening finger forming a third set of fingers, there would be a finger of the second set on each side of each finger of the first set, but not necessarily immediately adjacent thereto.

Each of the fastening projections 6 has a sharpened cutting edge 14 at its tip. Each edge is sharpened by virtue of continuation of the taper of the fastening finger on which the projection is located, and also a reduction in thickness of the fastening projection towards its tip.

The body portion 4 of the fixation device has a plurality of depressions 20 formed in it. which are directed inwardly which can engage the surface of a bone on which the device is to be used. The depressions are circular when viewed from above, and are perforated so that they can receive a fastener such as a screw. In the illustrated embodiment, the depressions are formed by punching so that there is a recess 22 in the plate, opposite to each projection on the surface that faces away from a bone when the fixation device is in use. The recess can accommodate the countersunk head of the fastener screw so that the screw presents a substantially flush surface with the body section.

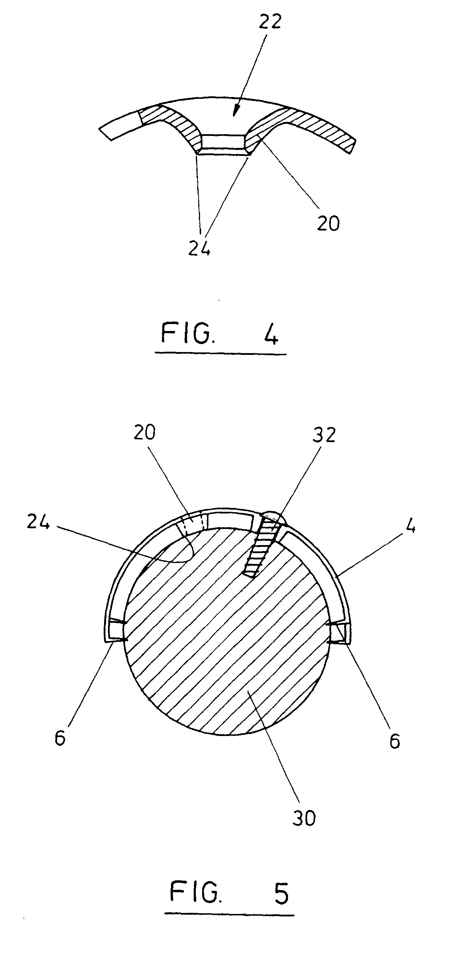

The edges 24 of the depressions which face towards the bone when in use are sharpened as can be seen in Figure 4.

The fixation device that is shown in the drawings can be formed from a sheet of a selected material by creating an appropriate profile using appropriate cutting techniques, and forming it into an arcuate configuration by bending. Cutting edges on the fastening projections can be formed by grinding techniques. For example, the cutting edges on the depressions can be formed by a rotating grinding tool. The depressions can be formed by a punching technique.

The dimensions of a device which might be used to fix a fracture in the femur, might be in the following ranges:

| Dimension | | Length (mm) |
|---|---|---|
| A | Length of device | 70 - 150 |
| B | Spacing between finger tips | 5 - 20 |
| C | Length of projections | 4 - 8 |
| D | Width of fingers at tips | 2 - 5 |
| E | Spacing between depressions | 18 - 40 |
| F | Diameter of depressions | 10 - 15 |
| G | Diameter of perforations | 3 - 5 |
| H | Internal transverse | 30 - 50 |

Figure 5 shows the fixation device in use, positioned around a fractured bone 30 so as to span the fracture. The body portion is positioned so that at least some of the depressions 20 are in contact with the surface of the bone. When so located, the side edges 7, 8 of the fixation device are forced inwardly towards one another so that the fastening projections 6 form an indented impression in the side surfaces of the bone. The wavy configuration of the line 10 which joins the fastening projections means that the fastening projections do not create a continuous straight line of weakness in the bone.

The fixation device is further secured to the bone by means of screws 32 inserted through the perforations in the depressions 20 in the body portion of the device. This can facilitate the formation of indented impressions in the surface of the bone by the cutting edges 14 of the depressions 20. The fixation device can then provide stability of the bone during healing of the fracture.

## Claims

1. A fixation device (2) for a bone fracture, which comprises a plate which is arcuate when viewed along its longitudinal axis so that it can be fitted around the bone in the region of the fracture, the plate having a body portion (4) and an array of inwardly directed fastening projections (6) along each of its opposite edges (6, 7) which can fixedly engage the bone along its length when the plate is positioned around the bone and its edges are subjected to an inward clamping force, the device having a plurality of depressions (20) formed in its body portion and extending inwardly towards the bone, in which at least some of the depressions can engage the surface of the bone to maintain a gap between the bone and the internal surface of the body portion in regions of the plate around the depressions **characterised in that**, the depressions are perforated (22) so that they can accommodate a fastener (32) by which the plate can be fixed to the bone.

2. A fixation device as claimed in claim 1, in which the depressions have a cutting edge which can form an indented impression in the surface of the bone when pressed against the surface.

3. A fixation device as claimed in claim 1 or claim 2, in which the depressions are arranged in at least two spaced apart linear arrays, the arrays extending along the device parallel to the axis thereof.

4. A fixation device as claimed in any one of claims 1 to 3, in which the projections are generally rounded when viewed from above.

5. A fixation device as claimed in any one of claims 1 to 5, in which the projections are formed by a punching operation to that there is a recess in the plate opposite to each projection.

6. A fixation device as claimed in any one of claims 1 to 5, in which the fastening projections are arranged so that lines extending along respective edges of the plate joining adjacent fastening projections each have a wavy configuration so that the lines each cross at several points respective references lines which extend along the device following the configuration of the body portion.

7. A fixation device as claimed in claim 6, in which the plate comprises two arrays of fastening fingers (9) extending generally around the longitudinal axis and arranged along the said opposite edges of the body portion, the fastening projections being provided on the fastening fingers.

8. A fixation device as claimed in claim 7, in which the length of the fastening fingers varies along the length of the device so that the line which joins adjacent fastening projections has a wavy configuration.

9. A fixation device as claimed in claim 8, in which the fastening fingers are arranged in first and second sets (11, 12), each fastening finger of the first set having a fastening finger of the second set on each side, the length of each fastening finger of the first set being less than the length of each of the adjacent fastening fingers of the second set.

10. A fixation device as claimed in claim 9, in which there is one fixation finger of the second set between each pair of adjacent fastening fingers of the first set.

11. A fixation device as claimed in claim 9 or claim 10, in which the lengths of fastening fingers of the first set are approximately equal.

12. A fixation device as claimed in any one of claims 9 to 11, in which the lengths of fastening fingers of the second set are approximately equal.

13. A fixation device as claimed in any one of claims 6 to 12, in which the fastening fingers are tapered inwardly towards their ends.

## Patentansprüche

1. Vorrichtung (2) zur Fixierung eines Knochenbruches, welche aus einer Platte besteht, die entlang ihrer Längsachse betrachtet gebogen ist, so daß sie im Bereich des Bruches rund um den Knochen an diesen angepaßt werden kann, wobei die Platte einen Grundkörper (4) sowie entlang ihrer einander gegenüberliegenden Kanten (6, 7) eine Anordnung nach innen gerichteter Befestigungsvorsprünge (6) aufweist, welche entlang der Länge des Knochens fest in diesen eingreifen, wenn die Platte rund um den Knochen positioniert wird und ihre Kanten einer nach innen gerichteten Klemmkraft ausgesetzt werden und wobei die Vorrichtung weiterhin eine Vielzahl von in ihrem Grundkörper geformten Vertiefungen (20) aufweist, die sich nach innen zum Knochen hin erstrecken und sich zumindest einige der Vertiefungen an der Oberfläche des Knochens anlegen, um einen Spalt zwischen dem Knochen und der Unterseite des Grundkörpers in den Bereichen der Platte rund um die Vertiefungen aufrechtzuerhalten, **dadurch gekennzeichnet, daß** die Vertiefungen gelocht (22) sind, so daß sie ein Befestigungselement (32) aufnehmen können, durch welches die Platte am Knochen befestigt werden kann.

2. Vorrichtung zur Fixierung nach Anspruch 1, bei welcher die Vertiefungen eine Schneidkante aufweisen, die eine gewollten Eindruck in der Oberfläche des Knochen formen kann, wenn die Vorrichtung gegen die Oberfläche gedrückt wird.

3. Vorrichtung zur Fixierung nach Anspruch 1 oder 2, bei welcher die Vertiefungen in mindestens zwei räumlich getrennten Linear-Anordnungen angeordnet sind, die sich parallel zur Achse der Vorrichtung an dieser entlang erstrecken.

4. Vorrichtung zur Fixierung nach einem der Ansprüche 1 bis 3, bei welcher die Vorsprünge von oben gesehen allgemein abgerundet sind.

5. Vorrichtung zur Fixierung nach einem der Ansprüche 1 bis 4, bei welcher die Vorsprünge durch einen Lochungsvorgang geformt werden, so daß in der Platte entgegengesetzt zum Vorsprung eine Vertiefung entsteht.

6. Vorrichtung zur Fixierung nach einem der Ansprüche 1 bis 5, bei welcher die Befestigungsvorsprünge derart angeordnet sind, daß Linien entlang der jeweiligen Kante der Platte, welche benachbarte Befestigungsvorsprünge verbinden, jeweils eine Wellenform haben, so daß diese Linien an einigen Punkten jeweilige Referenzlinien schneiden, welche sich entlang der Vorrichtung entsprechend der Gestalt des Grundkörpers erstrecken.

7. Vorrichtung zur Fixierung nach Anspruch 6, bei welcher die Platte zwei Reihen von Befestigungsfingern (9) aufweist, welche sich allgemein rund um die Längsachse erstrecken und entlang der einander gegenüberliegenden Kanten des Grundkörpers angeordnet sind, wobei die Befestigungsvorsprünge an den Befestigungsfingern vorgesehen sind.

8. Vorrichtung zur Fixierung nach Anspruch 7, bei welcher die Länge der Befestigungsfinger entlang der Länge der Vorrichtung derart variiert, daß die Linie, welche benachbarte Befestigungsvorsprünge verbindet, eine Wellenform hat.

9. Vorrichtung zur Fixierung nach Anspruch 8, bei welcher die Befestigungsfinger in einem ersten und einem zweiten Satz (11, 12) angeordnet sind, jeder Befestigungsfinger des ersten Satzes zu beiden Seiten einen Befestigungsfinger des zweiten Satzes hat und die Länge eines jeden Befestigungsfingers des ersten Satzes geringer ist als die Länge eines jeden benachbarten Befestigungsfingers des zweiten Satzes.

10. Vorrichtung zur Fixierung nach Anspruch 9, bei welcher sich ein Befestigungsfinger des zweiten Satzes zwischen einem Paar benachbarter Befestigungsfinger des ersten Satzes befindet. ,

11. Vorrichtung zur Fixierung nach Anspruch 9 oder 10, bei welcher die Länge der Befestigungsfinger des ersten Satzes annähernd gleich ist.

12. Vorrichtung zur Fixierung nach einem der Ansprüche 9 bis 11, bei welcher die Länge der Befestigungsfinger des zweiten Satzes annähernd gleich ist.

13. Vorrichtung zur Fixierung nach einem der Ansprüche 9 bis 11, bei welcher die Befestigungsfinger zu ihren Enden hin nach innen abgeschrägt sind.

## Revendications

1. Dispositif de fixation (2) d'un os fracturé, qui comprend une plaque qui est arquée telle qu'observée suivant son axe longitudinal, de sorte qu'il peut être ajusté autour de l'os dans la région de la fracture, la plaque ayant une partie de corps (4) et une rangée de saillies d'attache (6) orientées vers l'intérieur de l'arc depuis et le long de chacun de ses bords opposés (7, 8) qui peuvent s'engager de manière fixe sur la longueur de l'os quand la plaque est positionnée autour de l'os et que ses bords sont soumis à une force de serrage vers l'intérieur, le dispositif ayant une pluralité de cavités (20) formées dans sa partie de corps et s'étendant vers l'intérieur de l'arc en direction de l'os, dans lesquelles au moins certaines des cavités peuvent s'engager dans la surface de l'os de façon à laisser un espace entre l'os et la surface interne de la partie de corps dans les régions de la plaque autour des cavités, **caractérisé en ce que** les cavités sont perforées (22) de manière à pouvoir recevoir une attache (32) grâce à laquelle la plaque peut être fixée à l'os.

2. Dispositif de fixation selon la revendication 1, dans lequel les cavités ont un bord coupant qui peut former une empreinte creusée dans la surface de l'os suite à une pression contre la surface.

3. Dispositif de fixation selon la revendication 1 ou la revendication 2, dans lequel les cavités sont agencées en au moins deux rangées linéaires espacées, les rangées s'étendant le long du dispositif parallèlement à l'axe de celui-ci.

4. Dispositif de fixation selon l'une quelconque des revendications 1 à 3, dans lequel les saillies sont globalement arrondies telles qu'observées par le dessus.

5. Dispositif de fixation selon l'une quelconque des revendications 1 à 4, dans lequel les saillies sont formées par une opération de poinçonnage de façon qu'il y ait un évidement dans la plaque en face de chaque saillie.

6. Dispositif de fixation selon l'une quelconque des revendications 1 à 5, dans lequel les saillies d'attache sont agencées de telle sorte que les lignes s'étendant le long des bords respectifs de la plaque et joignant des saillies d'attache adjacentes ont chacune une configuration ondulée, de sorte que les lignes coupent chacune en plusieurs points des lignes de référence respectives qui s'étendent le long du dispositif en suivant la configuration de la partie de corps.

7. Dispositif de fixation selon la revendication 6, dans lequel la plaque comprend deux rangées de doigts d'attache (9) s'étendant globalement autour de l'axe longitudinal et agencés le long desdits bords opposés de la partie de corps, les saillies d'attache étant formées sur les doigts de maintien.

8. Dispositif de fixation selon la revendication 7, dans lequel la longueur des doigts d'attache varie tout le long du dispositif de telle manière que la ligne joignant des saillies d'attache adjacentes a une configuration ondulée.

9. Dispositif de fixation selon la revendication 8, dans lequel les doigts d'attache sont agencés en des premier et second jeux (11, 12), chaque doigt d'attache du premier jeu ayant un doigt d'attache du second jeu de chaque côté, la longueur de chaque doigt d'attache du premier jeu étant inférieure à la longueur de chacun des doigts d'attache adjacents du second jeu.

10. Dispositif de fixation selon la revendication 9, dans lequel il y a un doigt de fixation du second jeu entre chaque paire de doigts d'attache adjacents du premier jeu.

11. Dispositif de fixation selon la revendication 9 ou la revendication 10, dans lequel les longueurs des doigts d'attache du premier jeu sont approximativement égales.

12. Dispositif de fixation selon l'une quelconque des revendications 9 à 11, dans lequel les longueurs des doigts d'attache du second jeu sont approximativement égales.

13. Dispositif de fixation selon l'une quelconque des revendications 6 à 12, dans lequel les doigts d'attache sont fuselés vers l'intérieur en direction de leur extrémité.
